# EUROPÄISCHE PATENTANMELDUNG

(11) **EP 3 266 499 A1**
(43) Veröffentlichungstag der Anmeldung: **10.01.2018**
(21) Anmeldenummer: 17177070.4
(22) Anmeldetag: 21.06.2017
(51) Int. Cl.: A61N 1/362

(54) **ELEKTRODENFIXIERHÜLSE**

(30) Priorität: 04.07.2016 DE 102016112178
(71) Anmelder: BIOTRONIK SE & Co. KG, 12359 Berlin (DE)
(72) Erfinder: Ulmer, Jens, 8700 Küsnacht (CH)
(74) Vertreter: Keck, Hans-Georg

(57) **Zusammenfassung**

Die Erfindung betrifft eine Elektrodenfixierhülse (12) zur Fixierung einer Elektrodenleitung (16) an biologischem Gewebe, umfassend wenigstens einen Hülsenkörper (18), der wenigstens anteilig elastische Eigenschaften aufweist und der wenigstens einen Elektrodenführungsbereich (20) umfasst, wobei der Hülsenkörper (18) und der Elektrodenführungsbereich (20) eine gemeinsame Längsachse (L) aufweisen.

Erfindungsgemäß ist vorgesehen, dass der Hülsenkörper (18) wenigstens einen Fadengang (24) aufweist, der zumindest abschnittsweise entlang der gemeinsamen Längsachse (L) und helixartig um den Elektrodenführungsbereich (20) herum verläuft.

Weiterhin betrifft die Erfindung ein System (10) mit der Elektrodenfixierhülse (12) und einem chirurgischen Faden (14), sowie mit dem chirurgischen Faden (14) und einer Elektrodenleitung (16).

## Beschreibung

Gegenstände der Erfindung sind eine Elektrodenfixierhülse zur Fixierung einer Elektrodenleitung an biologischem Gewebe gemäß dem Oberbegriff des Patentanspruchs 1 und ein System gemäß dem Oberbegriff des Patentanspruchs 9 mit der Elektrodenfixierhülse.

### Technologischer Hintergrund und Stand der Technik

Herz-Kreislauf-Erkrankungen zählen zu den schwerwiegendsten Krankheiten der modernen Gesellschaft. Viele Erkrankungen verlaufen auch heute noch tödlich. Vor allem ältere Menschen sind von Herz-Kreislauf-Erkrankungen betroffen. Angesichts der steigenden Lebenserwartung und der wachsenden Anzahl chronischer Herzerkrankungen ist deshalb mit einer weiteren Zunahme dieser Krankheiten zu rechnen. Immer wieder angeführte Hauptursachen sind verbreitete Einflussfaktoren einer modernen und global vernetzten Gesellschaft wie Stress, Rauchen, zu fettes Essen, damit einhergehendes Übergewicht oder Bluthochdruck. Aber auch genetische Disposition oder Virusinfektionen können Herzerkrankungen verursachen. Da sich Störungen des Herz-Kreislauf-Systems bereits in jüngeren Jahren manifestieren und besonders jüngere Menschen von Beginn an in einer diese Einflussfaktoren begünstigenden Umwelt aufwachsen, ist mit einer weiteren Verstärkung des Trends zu Herz-Kreislauf-Erkrankungen zu rechnen. Einen wichtigen Ansatzpunkt stellt deshalb die konsequente Verbesserung der medizinischen Versorgung dar.

Dies führt zum einen zu steigenden Kosten und zum anderen auch zu höheren Anforderungen an die Sicherheit medizintechnischer Produkte, zum Beispiel bei Herzschrittmachern, da zukünftig tendenziell eine zunehmende Anzahl dieser Systeme im Umlauf sein wird und die Sicherheit gegen Störungen entsprechend groß sein muss.

Durch eine inhomogene Kontraktion der einzelnen Areale der linken Herzkammer kann eine Herzschwäche, auch Herzinsuffizienz genannt, entstehen. Hauptgrund für die inhomogene, beziehungsweise asynchrone Kontraktion ist eine Störung im Reizleitungssystem des Herzens. Durch die sogenannte Cardiac Resynchronization Therapy, kurz CRT-Stimulation, wird die Kontraktion der linken Herzkammer wieder homogen, vereinfacht ausgedrückt resynchronisiert, und die Pumpkraft des Herzens kann sich wieder erholen. Das Verfahren sieht die Verwendung von zwei Elektroden vor. Eine Elektrode wird in die rechte Herzkammer implantiert. Das Zielgefäß für die, die linke Herzkammer betreffende, Elektrode ist der Coronarsinus. So wird das Gefäß bezeichnet, welches das venöse Blut aus den Herzkranzgefäßen in den rechten Vorhof leitet. Die Elektrode wird in einen Seitenast des Coronarsinus implantiert und liegt auf der Außenseite der linken Herzkammer. Durch die simultane Impulsabgabe über beide Elektroden wird der linke Teil des Herzens wieder synchron elektrisch erregt und eine homogene Kontraktion ist möglich.

Die Befestigung der elektrischen Leitungen zu den Elektroden erfolgt über sogenannte Elektrodenfixierhülsen (EFH). Darunter versteht man eine spezielle Fixierhülse, die an einer definierten Stelle fest auf der Elektrodenleitung angeordnet wird und die dann an geeigneter Stelle im menschlichen Körper befestigt wird, zum Beispiel durch Annähen im Bereich eines Muskels oder eines Gefäßes. Bei neueren Varianten werden spezielle Fixierhülsen verwendet, die zusammengepresst oder festgeschraubt werden und dann eine feste Einheit mit der Elektrode bilden. Häufig sind solche Elektrodenfixierhülsen aus einem weichen Kunststoff hergestellt und werden über die Elektrodenleitung geschoben. Mit auch Ligatur genannten Nähfaden wird dann eine Fixierungskraft auf die Elektrodenfixierhülse aufgebracht und diese dadurch an die Elektrodenleitung angepresst. Durch die entstehende Haftreibung wird die Elektrodenfixierhülse axial auf der Elektrodenleitung fixiert. Die Elektrodenfixierhülse kann dann an geeigneter Stelle im Körper vernäht werden.

Als problematisch erweist sich hierbei, dass es bei der manuellen Anbringung der Ligatur zu einer Beschädigung der Elektrodenleitung kommen kann, wenn die aufgebrachte Fixierungskraft zu hoch ist. In einem anderen Fall kann hingegen eine ungewollte Verlagerung der Elektrodenleitung auftreten, wenn die aufgebrachte Fixierungskraft zu gering ist.

Die EP 1 933 934 B1 offenbart eine Nahthülse zum Befestigen einer implantierbaren Leitung an Körpergewebe. Die Schrift schlägt vor, mit Hilfe eines Verriegelungsmechanismus eine Beschädigung der Elektrodenleitung zu vermeiden.

Weiterhin beschreibt die US 4,553,961 eine Nahthülse mit einer Haftungsverbesserungsstruktur, die in der Nahthülse angeordnet ist und beispielsweise zusätzliche zahnartige Strukturen aufweist, mit denen die Elektrodenleitung sicher fixiert werden kann. Die Haftungsverbesserungsstruktur bildet gleichzeitig einen Schutz gegen eine zu starke Krafteinwirkung auf die Elektrodenleitung durch die Ligatur.

Die US 5,824,032 zeigt eine Verankerungshülse zur Befestigung einer Elektrodenleitung in menschlichem Gewebe. Zur Befestigung der Elektrodenleitung in der Verankerungshülse kommt eine Art Lasche zum Einsatz, die ausgebildet ist, die Elektrodenleitung zu umschlingen und durch Verriegelung der Lasche zu sichern.

Ferner offenbart die US 8,271,096 B2 eine Elektrodenfixierhülse aus einem elastischen Material mit einem darin eingeordneten formstabilen Kompressionsregler.

Der Erfindung liegt nun die Aufgabe zugrunde, eine alternative Elektrodenfixierhülse in einfacher Bauart zu schaffen, mit der eine lokale Beschädigung der Elektrodenleitung vermieden wird, wie sie bei konventionellen Elektrodenfixierhülsen infolge der übertragenen Fixierungskraft häufig auftritt. Dabei soll stets eine sichere Fixierung der Elektrodenleitung gewährleistet sein.

Die Aufgabe wird durch die Gegenstände mit den Merkmalen der Patentansprüche 1 und 9 gelöst.

Ein erster Aspekt der Erfindung betrifft eine Elektrodenfixierhülse zur Fixierung einer Elektrodenleitung an biologischem Gewebe, umfassend wenigstens einen Hülsenkörper, der wenigstens anteilig elastische Eigenschaften aufweist und der wenigstens einen Elektrodenführungsbereich umfasst, wobei der Hülsenkörper und der Elektrodenführungsbereich eine gemeinsame Längsachse aufweisen. Erfindungsgemäß ist vorgesehen, dass der Hülsenkörper wenigstens einen Fadengang aufweist, der zumindest abschnittsweise entlang der gemeinsamen Längsachse und helixartig um den Elektrodenführungsbereich herum verläuft.

In den Fadengang kann zur Fixierung der Elektrodenleitung, wenn diese in dem Elektrodenführungsbereich angeordnet ist, ein chirurgischer Faden eingebracht werden. Durch Aufbringen einer Zugkraft auf den chirurgischen Faden kann dann eine Fixierungskraft auf die Elektrodenleitung aufgebracht werden, die einen Haftschluss zwischen der Elektrodenleitung und dem Elektrodenführungsbereich herbeizuführen vermag.

Mit anderen Worten ausgedrückt kann die Elektrodenfixierhülse einen länglichen Hülsenkörper aufweisen, in dessen Längsrichtung eine Elektrodenleitung durch den länglichen Hülsenkörper hindurchführbar ist. Durch radiales Zusammendrücken des Hülsenkörpers kann zwischen der Elektrodenleitung und der Elektrodenfixierhülse ein Haftschluss ausgebildet werden. Da der Hülsenkörper elastisch ist, ist der Haftschluss reversibel ausbildbar. Im Sinne der Erfindung ist ein Zusammendrücken des Hülsenkörpers insbesondere mittels eines chirurgischen Fadens vorgesehen. Dieser kann hierzu in einem ersten Schritt mit der Elektrodenfixierhülse zusammen oder getrennt bereitgestellt werden. Der chirurgische Faden kann dann durch den Fadengang sowohl entlang des länglichen Hülsenkörpers, als auch mehrfach herumlaufend um jenen Bereich geführt werden, in dem die Elektrodenleitung durch den länglichen Hülsenkörper führbar ist. Zur Führung des chirurgischen Fadens weist der längliche Hülsenkörper einen entsprechend helixartig ausgebildeten Fadengang auf, der als Hohlraum in dem länglichen Hülsenkörper hergestellt ist.

Der Fadengang kann, bei beispielsweise direkt im Zuge eines Urformungsprozesses in den Hülsenkörper eingebracht werden. Der Hülsenkörper kann beispielsweise aus Silikon bestehen. Der Hülsenkörper kann aber auch unter Verwendung eines Copolymers, bestehend aus Urethane und Silicone hergestellt werden. Weitere Werkstoffe sind Copolyamide (Pebax), Styrol, Blockcopolymere (SBS, SEBS, SEPS, SEEPS, MBS), Olefin basierte Elastomere sowie thermoplastische Polyester. Beispielsweise kann der Hülsenkörper gegossen oder spritzgegossen werden. Dabei kann ein den Fadengang repräsentierender Kern eingebracht werden, der eine entsprechende helixartige Form aufweist. Anschließend kann der Kern, begünstigt durch seine helixartige Form, gewissermaßen aus dem Hülsenkörper herausgeschraubt werden. Der Hülsenkörper kann aber auch mehrteilig hergestellt werden, wobei unterschiedliche Teile nach dem Urformen zusammengefügt werden. Weitere Herstellungsverfahren erschließen sich dem zuständigen Fachmann ohne weiteres.

Die erfindungsgemäße Elektrodenfixierhülse bietet eine Reihe an Vorteilen. So kann die auf die Elektrodenleitung übertragbare Fixierungskraft über den Fadengang, da dieser helixartig verläuft, besonders homogen verteilt werden, wenn die Zugkraft auf den chirurgischen Faden wirkt. Die Fixierungskraft ist dann homogen, sowohl radial um die Elektrodenleitung herum auf diese übertragbar, als auch entlang der gemeinsamen Längsachse verteilt übertragbar. Somit ist ein sicherer Haftschluss zwischen der Elektrodenleitung und dem Elektrodenführungsbereich herstellbar. Gleichzeitig ist eine zu starke lokale Belastung der Elektrodenleitung vermeidbar. Bezogen auf die lokalen Abschnitte der Elektrodenleitung erleichtert dies auch eine Begrenzung der Fixierungskraft. So ist im Zuge einer kinetischen Umwandlung der Zugkraft in die Fixierungskraft, die im Wesentlichen eine radial in Richtung der gemeinsamen Längsachse aufbringbare Kraft ist, eine Übersetzung. Somit ist in den lokalen Bereichen bei Wirksamkeit einer relativ großen Zugkraft eine relativ kleine Fixierungskraft wirksam. Dies ist eine Folge der homogenen Kraftverteilung über eine größere Haftfläche. Hierdurch sind größere Toleranzen hinsichtlich der zulässigen maximalen Zugkraft am chirurgischen Faden erlaubt. Dies erhöht die Sicherheit gegen ein zu festes, beispielsweise manuelles, Anziehen des chirurgischen Fadens signifikant. Die genaue kinematische und kinetische Auslegung der Zugkraft und der Fixierungskraft nimmt der Fachmann unter allgemein bekannten Grundsätzen der technischen Mechanik und der Werkstofftechnik selbstständig vor. Dabei berücksichtigt er insbesondere eine maximale Belastbarkeit der der Auslegung zugrunde gelegten Elektrodenleitung. Die Elektrodenfixierhülse wird, für sich allein betrachtet, anhand einer theoretischen Elektrodenleitung ausgelegt und ist später mit dieser Elektrodenleitung verwendbar. Ferner berücksichtigt der Fachmann eine für den Haftschluss benötigte minimal übertragbare Haftreibungskraft und die eine maximal auf den chirurgischen Faden aufbringbare Zugkraft. Weitere allgemeine Auslegungsparameter identifiziert der Fachmann aufgrund seines Fachwissens ohne weiteres.

In bevorzugter Ausgestaltung der Elektrodenfixierhülse der Erfindung ist vorgesehen, dass der Fadengang sich entlang der gemeinsamen Längsachse von einem proximalen Ende des Hülsenkörpers bis zu einem distalen Ende des Hülsenkörpers erstreckt. Mit anderen Worten lässt sich der chirurgische Faden entlang des gesamten Hülsenkörpers in dem Fadengang führen.

Dies bietet den Vorteil, dass eine möglichst große Haftfläche zur Übertragung der Fixierungskraft zur Verfügung steht und die Fixierungskraft noch besser und gleichmäßiger auf eine Elektrodenleitung übertragbar ist.

In weiterer bevorzugter Ausgestaltung der Elektrodenfixierhülse der Erfindung ist vorgesehen, dass der Fadengang in einem konstanten radialen Abstand von der gemeinsamen Längsachse um den Elektrodenführungsbereich herum verläuft. Betrachtet man den Fadengang als geometrisch ideale Helix, so entspricht die Helix in dieser Ausführungsform einer Helix mit konstant bleibendem Durchmesser. Die gemeinsame Längsachse wäre hier gleichzusetzen mit einer Längsachse der Helix. Der radiale Abstand wird stets ausgehend von einem Raummittelpunkt des Fadengangs als Strecke senkrecht auf die gemeinsame Längsachse betrachtet. Zur Definition des Raummittelpunkts wird der Fadengang hilfsweise als räumlich ausgetragener Ausschnitt aus dem Hülsenkörper betrachtet. Dieser ausgetragene Ausschnitt ist modellierbar durch eine Grundfläche und eine Austragungsbahnkurve, entlang derer die Grundfläche bewegt wird und so ein Volumen aus dem Fadengang austrägt. Dem Fachmann ist diese Modellbetrachtung aus dem Bereich der CAD-Konstruktion gut bekannt. Der für die Bestimmung des radialen Abstands betrachtete Raummittelpunkt liegt dabei stets in einem Punkt, in dem die Grundfläche von der Austragungsbahnkurve geschnitten wird.

Der konstante radiale Abstand bietet den Vorteil, dass die Elektrodenfixierhülse besonders einfach herstellbar ist. Besonders vorteilhaft ist dies bei der Verwendung eines Kerns im Urformprozess, der anschließend aus dem Hülsenkörper herausgeschraubt werden soll.

In weiterer bevorzugter Ausgestaltung der Elektrodenfixierhülse der Erfindung ist vorgesehen, dass der Fadengang in einem wenigstens einmal variierenden radialen Abstand von der gemeinsamen Längsachse um den Elektrodenführungsbereich herum verläuft. Betrachtet man den Fadengang wiederum als Helix, so entspricht dies einer Helix mit einem wenigstens einmalig veränderten Durchmesser. Ohnehin wird der Begriff der Helix im Zusammenhang mit der Erfindung hilfsweise und nicht beschränkt auf eine geometrisch ideale Helix verwendet. So können beispielsweise der Durchmesser oder eine Gangsteigung der Helix variieren.

Diese Ausgestaltung bietet den Vorteil, dass die Fixierungskraft durch Variation des radialen Abstands bei konstanter Zugkraft am chirurgischen Faden lokal variiert werden kann. Beispielsweise kann durch abschnittsweise Reduktion des radialen Abstands, also durch abschnittsweise Reduktion des Durchmessers der Helix, die abschnittsweise auf die Elektrodenleitung übertragbare Fixierungskraft erhöht werden. Analog kann die übertragbare Fixierungskraft durch Erhöhung des radialen Abstands beziehungsweise des Durchmessers bei gleichbleibender Zugkraft verringert werden.

Beispielsweise kann der radiale Abstand des Fadengangs von der gemeinsamen Längsachse in Richtung des distalen Endes des Hülsenkörpers abschnittsweise kleiner sein, als in Richtung des proximalen Endes. Somit ergibt sich in einem theoretischen Modell ohne Reibungseinfluss (beziehungsweise ohne Einfluss dissipativer Kräfte) in Richtung des distalen Endes abschnittsweise eine höhere Fixierungskraft, als in Richtung des proximalen Endes. In der Realität hingegen ist der Reibungseinfluss selbstverständlich vorhanden. Dies führt in der Realität dazu, dass in Abhängigkeit einer Wegstrecke, die der chirurgische Faden in dem Fadengang zurücklegt, Energie aufgrund des Reibungseinflusses in eine Innenwand des Fadengangs eingetragen wird. Dies führt über die Wegstrecke zu einer Abnahme der noch verfügbaren Zugkraft und somit der Fixierungskraft. Die Wegstrecke wird ausgehend vom proximalen Ende des Hülsenkörpers betrachtet. In der Regel ist wenigstens dort die Zugkraft in den chirurgischen Faden einbringbar, beispielsweise durch einen Chirurgen, der den chirurgischen Faden handhabt. Die beschriebene Variation des radialen Abstands, die in der reibungsfreien Theorie eine inhomogene Verteilung der Fixierungskraft zur Folge hätte, kann nun so angepasst werden, dass die Reibungsverluste in der Realität vorteilhaft ausgeglichen werden können. Dies erfolgt durch die beschriebene Erhöhung des radialen Abstands des Fadengangs von der gemeinsamen Längsachse in Richtung des proximalen Endes und dessen Verringerung in Richtung des distalen Endes. Somit lässt sich unter Wirksamkeit der Reibungseinflüsse vorteilhaft eine homogene Verteilung der Fixierungskraft realisieren. Dies ist insbesondere auch bei längeren Hülsenkörpern von Nutzen, da die Reibungsverluste hier besonders groß sind.

In weiterer bevorzugter Ausgestaltung der Elektrodenfixierhülse der Erfindung ist vorgesehen, dass die Elektrodenfixierhülse eine zylindrische Durchgangsbohrung entlang der gemeinsamen Längsachse umfasst, die einen Durchmesser von 1 mm bis 3 mm aufweist. Bevorzugt beträgt der Durchmesser 1,3 mm bis 1,6 mm, weiterhin bevorzugt 1,8 mm bis 1,9 mm, und weiterhin bevorzugt 2,2 mm bis 2,6 mm.

Dies bietet den Vorteil, dass die Elektrodenfixierhülse insbesondere für alle in der Praxis üblichen Durchmesser von Elektrodenleitungen sehr gut geeignet ist.

In weiterer bevorzugter Ausgestaltung der Elektrodenfixierhülse der Erfindung ist vorgesehen, dass der radiale Abstand des Fadengangs von der gemeinsamen Längsachse derart gewählt ist, dass sich zwischen einer inneren Mantelfläche der zylindrischen Durchgangsbohrung und dem Fadengang ein konstanter oder variierender radialer Teilabstand von 0,5 mm bis 3 mm ergibt. Bevorzugt beträgt der radialer Teilabstand 1 mm bis 2 mm, weiterhin bevorzugt 1,3 mm bis 1,7 mm und weiterhin bevorzugt 1,5 mm. Der radiale Teilabstand stellt eine Teilstrecke innerhalb derselben Strecke dar, die zur Bestimmung des weiter oben beschriebenen radialen Abstands verwendet wird. Betrachtet wird die Teilstrecke ausgehend von einem Schnittpunkt der inneren Mantelfläche mit dieser Strecke, hin zu dem Raummittelpunkt des Fadengangs.

Experimentelle Untersuchungen haben ergeben, dass eine verbleibende Materialstärke des Hülsenkörpers zwischen einzelnen Windungen des Fadengangs und der gemeinsamen Längsachse somit stets noch eine sichere Übertragbarkeit der Fixierungskraft gewährleistet und gleichzeitig eine kompakte Baugröße der Elektrodenfixierhülse gegeben ist.

In weiterer bevorzugter Ausgestaltung der Elektrodenfixierhülse der Erfindung ist vorgesehen, dass der Fadengang einen Gangdurchmesser von 0,015 mm bis 1,5 mm und/oder eine Gangsteigung von 0,05 mm bis 3,02 mm pro Umlauf des Fadengangs beziehungsweise Windung um die gemeinsame Längsachse aufweist. Bevorzugt liegt der Gangdurchmesser bei 0,025 mm bis 1,4 mm, weiterhin bevorzugt bei 0,035 mm bis 1,2 mm, weiterhin bevorzugt bei 0,045 mm bis 1 mm, weiterhin bevorzugt bei 0,055 mm bis 0,95 mm, weiterhin bevorzugt bei 0,075 mm bis 0,85 mm, weiterhin bevorzugt bei 0,15 mm bis 0,75 mm, weiterhin bevorzugt bei 0,25 mm bis 0,65 mm, weiterhin bevorzugt bei 0,35 mm bis 0,55 mm und weiterhin bevorzugt bei 0,45 mm bis 0,5 mm. Vorzugsweise entspricht der minimale Wert der Gangsteigung dem Wert des doppelten Gangdurchmessers. Hinzugerechnet wird noch eine Wandstärke von 0,02 mm zwischen den Windungen.

Dies bietet den Vorteil, dass der Gangdurchmesser für alle in der Praxis gängigen chirurgischen Fäden gut geeignet ist und diese in dem Fadengang führbar sind, ohne dass Haftreibung entsteht. Mit anderen Worten sind also Gleitreibungsbedingungen realisierbar. Durch die Gangsteigung ist sichergestellt, dass eine verbleibende Materialstärke zwischen einzelnen Windungen des Fadengangs stets noch ausreicht, damit die Zugkraft über den chirurgischen Faden sicher übertragbar ist. Ferner vorteilhaft wird durch die Gangsteigung ein Auftreten von Haftreibung zusätzlich gehemmt.

In weiterer bevorzugter Ausgestaltung der Elektrodenfixierhülse der Erfindung ist vorgesehen, dass der Elektrodenführungsbereich einen Reibungskoeffizienten von wenigstens 0,8 gegenüber Kunststoffen aufweist. Vorzugsweise handelt es sich bei den für die Auslegung des Elektrodenführungsbereichs betrachteten Kunststoffen um Werkstoffe, die für Mantelschichten von Elektrodenleitungen verwendbar sind. Beispiele hierfür sind Silicone, Polyurethane, Copolymere und auch Copolyamide (PEBAX). Weitere Beispiele sind Urethane, Styrol, Blockcopolymere (SBS, SEBS, SEPS, SEEPS, MBS), Olefin basierte Elastomere sowie thermoplastische Polyester.

Dies bietet den Vorteil, dass ein Haftschluss zwischen einer Elektrodenleitung und dem Elektrodenführungsbereich besonders gut realisierbar ist.

Ein zweiter Aspekt der Erfindung betrifft ein System, wenigstens umfassend eine erfindungsgemäße Elektrodenfixierhülse und einen chirurgischen Faden.

Die Elektrodenfixierhülse des erfindungsgemäßen Systems und der chirurgische Faden entsprechend der obigen Beschreibung. Dies gilt auch für die oben beschriebenen konstruktiven Zusammenhänge und realisierbaren Wechselwirkungen zwischen der Elektrodenfixierhülse und dem chirurgischen Faden.

Das System der Erfindung bietet den Vorteil, dass mit dem chirurgischen Faden die Fixierungskraft homogen und sicher über die Elektrodenfixierhülse auf eine Elektrodenleitung übertragbar ist, wenn die Elektrodenleitung in dem Elektrodenführungsbereich der Elektrodenfixierhülse angeordnet ist. Alle weiteren Vorteile ergeben sich im Detail aus der obigen Beschreibung.

In bevorzugter Ausgestaltung des Systems der Erfindung ist vorgesehen, dass ein Gangdurchmesser und/oder eine Gangsteigung eines Fadengangs eines Hülsenkörpers der Elektrodenfixierhülse und ein Fadendurchmesser des chirurgischen Fadens und/oder deren Werkstoffe derart aufeinander abgestimmt sind, dass der chirurgische Faden unter Gleitreibungsbedingungen in dem Fadengang beweglich ist, wenn eine Zugkraft von 2 N bis 50 N auf den chirurgischen Faden wirkt.

Durch Wahl entsprechender Werkstoffe des chirurgischen Fadens und des Hülsenkörpers können Gleitreibungsbedingungen zusätzlich gefördert werden. Geeignete Kombinationen sind hier beispielsweise Silikon für den Hülsenkörper und Polyester oder Polypropylen für den chirurgischen Faden. Da es sich bei Silikonen um Werkstoffe handelt, die einen hohen Reibkoeffizienten besitzen, macht es hier Sinn, noch eine Beschichtung hinzuzufügen, die die Reibung reduziert. Bewährt hat sich hier die sogenannte Silglide-Technologie bei der Tetramethyldisiloxan in der Gasphase mit Hilfe eines Plasmas abgeschieden wird. Weitere Kombinationen zur Unterstützung der Gleitreibungsbedingungen legt der zuständige Fachmann anhand seiner Kenntnisse der Werkstofftechnik selbständig fest.

In weiterer bevorzugter Ausgestaltung des Systems der Erfindung ist vorgesehen, dass der chirurgische Faden an einem distalen Ende eines Hülsenkörpers der Elektrodenfixierhülse fest mit dieser verbunden ist. In solch einem Fall kann der chirurgische Faden jeweils formschlüssig, stoffschlüssig, kraftschlüssig oder mittels einer Kombination dieser Verbindungsprinzipien an dem Hülsenkörper befestigt sein.

Dies bietet den Vorteil, dass die Zugkraft lediglich an einem Ende des chirurgischen Fadens manuell aufzubringen ist und dieser an seinem anderen Ende von dem Hülsenkörper selbst gehalten wird. Ferner lässt sich der chirurgische Faden mit dem Hülsenkörper vorteilhaft als vormontierte Einheit bereitstellen.

In weiterer bevorzugter Ausgestaltung des Systems der Erfindung ist vorgesehen, dass das System weiterhin wenigstens eine Klemmhülse zur Festlegung des chirurgischen Fadens an einem proximalen Ende eines Hülsenkörpers der Elektrodenfixierhülse umfasst. Unter Aufbringung der Zugkraft kann die Klemmhülse über den chirurgischen Faden bis an einen Eingang des Fadengangs, beispielsweise am proximalen Ende des Hülsenkörpers, herangeführt werden und die Klemmhülse dann beispielsweise gekrimpt werden. Die Klemmhülse verhindert dann ein vollständiges Hineinrutschen des chirurgischen Fadens in den Fadengang und hält den chirurgischen Faden so in Position.

Dies bietet den Vorteil, dass der chirurgische Faden einfach, schnell und sicher an der Elektrodenfixierhülse fixierbar ist. Alternativ kann beispielsweise ein chirurgischer Knoten gesetzt werden.

In weiterer bevorzugter Ausgestaltung des Systems der Erfindung ist vorgesehen, dass System weiterhin wenigstens eine Elektrodenleitung umfasst.

Die Elektrodenleitung des erfindungsgemäßen Systems entspricht der obigen Beschreibung. Dies gilt auch für die oben beschriebenen konstruktiven Zusammenhänge und realisierbaren Wechselwirkungen zwischen der Elektrodenleitung und der Elektrodenfixierhülse sowie dem chirurgischen Faden.

Das System der Erfindung bietet in dieser Ausgestaltung den Vorteil, dass mit dem chirurgischen Faden eine Fixierungskraft homogen und sicher über die Elektrodenfixierhülse auf die Elektrodenleitung übertragen werden kann. Alle weiteren Vorteile ergeben sich im Detail aus der obigen Beschreibung.

In weiterer bevorzugter Ausgestaltung des Systems der Erfindung ist vorgesehen, dass ein Reibungskoeffizient zwischen dem Elektrodenführungsbereich des Hülsenkörpers der Elektrodenfixierhülse und der Elektrodenleitung in einem Bereich 0,8 oder darüber liegt.

Dies bietet den Vorteil, dass ein Haftschluss zwischen der Elektrodenleitung und dem Elektrodenführungsbereich besonders gut und sicher realisierbar ist.

In weiterer bevorzugter Ausgestaltung des Systems der Erfindung ist vorgesehen, dass der chirurgische Faden die Elektrodenfixierhülse und die Elektrodenleitung derart aufeinander abgestimmt sind, dass unter Wirksamkeit einer Zugkraft von 2N bis 50N auf den chirurgischen Faden ein Haftschluss zwischen dem Elektrodenführungsbereich der Elektrodenfixierhülse und der Elektrodenleitung herstellbar ist, der eine Haftreibungskraft von 2 N bis 50 N zu übertragen vermag. Bevorzugt beträgt die übertragbare Haftreibungskraft 5 N bis 25 N, weiterhin bevorzugt 7 N bis 10 N.

Dies bietet den Vorteil, dass die Elektrodenleitung mit der Elektrodenfixierhülse einfach und sicher fixiert werden kann. Die Zugkraft kann in den genannten Größenordnungen einfach und genau von Hand aufgebracht werden.

Zu Aufbringung einer sehr genau definierten Zugkraft kann das System zusätzlich ein Rastwerkzeug umfassen. Das Rastwerkzeug ist ausgebildet, den chirurgischen Faden und die Klemmhülse voneinander unabhängig einzuspannen und unter Festhalten der Klemmhülse eine definierte Zugkraft auf den chirurgischen Faden aufzubringen, so dass der chirurgische Faden in der Klemmhülse verlagerbar, beziehungsweise durch diese hindurchziehbar ist. Ferner weist das Rastwerkzeug einen Mechanismus auf, der bei Erreichen der definierten Zugkraft die Klemmhülse automatisch zu deformieren vermag. Beispielsweise kann der Mechanismus ausgebildet sein, die Klemmhülse zu krimpen. Beispielsweise kann der Mechanismus eine Spannfeder umfassen, die unter Wirksamkeit der Zugkraft gelängt oder gestaucht wird und die bei Erreichen der definierten Zugkraft einen weiteren Mechanismus zu krimpen der Klemmhülse freigibt.

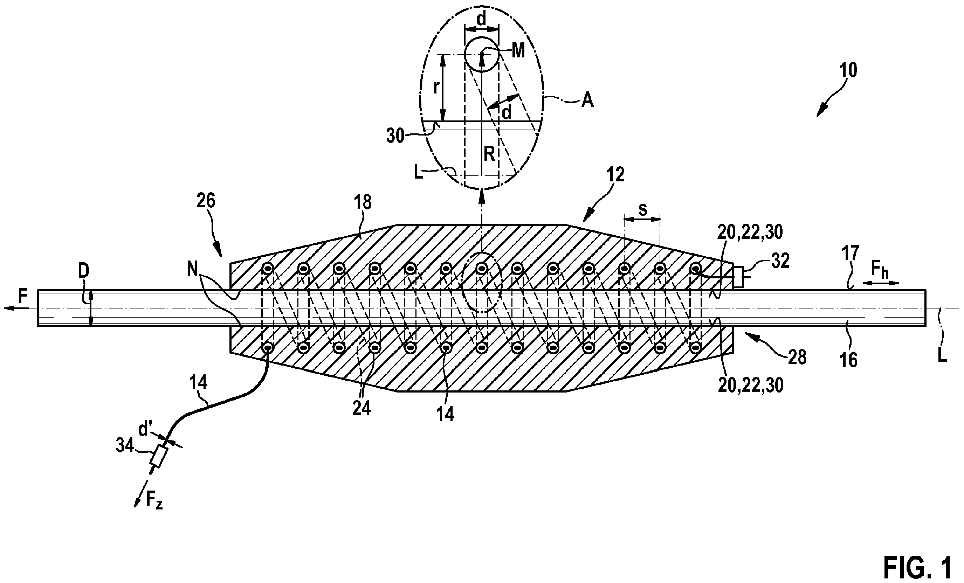

- Fig. 1: zeigt ein erfindungsgemäßes System mit einer erfindungsgemäßen Elektrodenfixierhülse und einem chirurgischen Faden sowie einer Elektrodenleitung in einer bevorzugten Ausführungsform.

Figur 1 zeigt ein erfindungsgemäßes System 10 mit einer erfindungsgemäßen Elektrodenfixierhülse 12, einem chirurgischen Faden 14 und einer Elektrodenleitung 16. Die Elektrodenfixierhülse 12 ist in einer Querschnittsansicht gezeigt. Die Elektrodenfixierhülse 12 umfasst wenigstens einen Hülsenkörper 18. Dieser besteht hier aus Silikon und weist somit elastische Eigenschaften auf. Die Elektrodenfixierhülse 12 weist an dem Hülsenkörper 18 weiterhin einen Elektrodenführungsbereich 20 auf. Der Hülsenkörper 18 insgesamt und der Elektrodenführungsbereich 20 weisen eine gemeinsame Längsachse L auf. Der Elektrodenführungsbereich 20 ist hier rein exemplarisch als zylindrische Durchgangsbohrung 22 ausgebildet. Diese erstreckt sich entsprechend entlang der gemeinsamen Längsachse L und weist rein exemplarisch einen Durchmesser D von 1,5 mm auf.

Ein wesentlicher Aspekt der Erfindung ist, dass der Hülsenkörper 18 einen Fadengang 24 aufweist, der entlang der gemeinsamen Längsachse L verläuft und dabei helixartig um den Elektrodenführungsbereich 20 herum verläuft. Der Fadengang 24 erstreckt sich entlang der gesamten gemeinsamen Längsachse L von einem proximalen Ende 26 des Hülsenkörpers 18 bis zu einem distalen Ende 28 des Hülsenkörpers 18. Detail A zeigt den Fadengang 24 in einer vergrößerten Ansicht und ohne Darstellung des chirurgischen Fadens 14. Der Fadengang 24 weist einen radialen Abstand R von der gemeinsamen Längsachse L auf, bezogen auf seinen Raummittelpunkt M. Entlang der gemeinsamen Längsachse L kann der radiale Abstand R sowohl konstant sein, wie in dem gezeigten Ausführungsbeispiel, als auch variabel. Durch den radialen Abstand R ergibt sich zwischen einer inneren Mantelfläche 30 der zylindrischen Durchgangsbohrung 22, die als Haftfläche dient, und dem Fadengang 24 entsprechend ein konstanter oder variierender radialer Teilabstand r. Dieser beträgt hier rein exemplarisch 1,5 mm. Ein Gangdurchmesser d des Fadengangs 24 beträgt hier rein exemplarisch 1,2 mm. Der Fadengang 24 weist auch eine definierte Gangsteigung s auf.

Dieser beträgt vorliegend exemplarisch bis zu 2,5 mm pro Umlauf des Fadengangs 24 um die gemeinsame Längsachse L. Wie vorliegend gezeigt, kann die Gangsteigung s abschnittsweise auch zu Null werden. Sie kann lokal somit konstant oder variabel entlang der gemeinsamen Längsachse L sein. Der Elektrodenführungsbereich 20 weist an der inneren Mantelfläche 30 einen Reibungskoeffizienten µ von 0,8 bis 1,2 gegenüber einem Werkstoff einer äußeren Mantelschicht 17 der Elektrodenleitung 16 auf. Der Reibungskoeffizient µ kann aber auch darüber oder darunter liegen und lässt sich beispielsweise durch Auftrag eines Gleitmittels beeinflussen. Der Zuständige Fachmann legt dies anhand der offenbarten Auslegungsaspekte selbstständig fest.

Figur 1 zeigt weiterhin den chirurgischen Faden 14 als Element des Systems 10. Dieser weist hier rein exemplarisch einen Fadendurchmesser d' von 1 mm auf. Er besteht in diesem Ausführungsbeispiel aus einem geflochtenen Multifilament Polyesterfaden.

Figur 1 zeigt weiterhin die Elektrodenleitung 16 als Element des Systems 10. Diese weist hier denselben Durchmesser D auf, wie die zylindrische Durchgangsbohrung 22. Die Elektrodenleitung 16 und die zylindrische Durchgangsbohrung 22 bilden vorzugsweise ein Passungssystem, das ohne Einwirkung einer Fixierungskraft eine enge Spielpassung darstellt. Vorliegend besteht die äußere Mantelschicht 17 der Elektrodenleitung 16 aus Silicon.

Durch die beschriebenen beispielhaften Ausprägungen der Merkmale des Fadengangs 24, des Hülsenkörpers 18, des chirurgischen Fadens 14 und der Elektrodenleitung 16 wird Folgendes erreicht: Der Fadengang 24 des Hülsenkörpers 18 der Elektrodenfixierhülse 12 und der chirurgische Faden 14 sind zum einen derart aufeinander abgestimmt, dass der chirurgische Faden 14 unter Gleitreibungsbedingungen in dem Fadengang 24 beweglich ist, wenn eine Zugkraft F_{z} von bis zu 50 N auf den chirurgischen Faden 14 wirkt. Zum anderen sind der Elektrodenführungsbereich 20 des Hülsenkörpers 18 der Elektrodenfixierhülse 12 und die äußere Mantelschicht 17 der Elektrodenleitung 16 derart aufeinander abgestimmt, dass unter Wirksamkeit der Zugkraft F_{z}, sobald diese mit mindestens 20 N auf den chirurgischen Faden 14 wirkt, ein sicherer Haftschluss zwischen dem Elektrodenführungsbereich 20 der Elektrodenfixierhülse 12 und der äußeren Mantelschicht 17 der Elektrodenleitung 16 vorliegt. In diesem Ausführungsbeispiel vermag dieser Haftschluss eine Haftreibungskraft Fₕ von bis zu 10 N zu übertragen.

In dem vorliegenden Ausführungsbeispiel ist der chirurgische Faden 14 an dem distalen Ende 28 des Hülsenkörpers 18 der Elektrodenfixierhülse 12 fest mit dieser verbunden. Hierzu ist exemplarisch eine distale Klemmhülse 32 gezeigt. An dem proximalen Ende 26 ist ebenfalls eine Klemmhülse 34 gezeigt, die sich über den chirurgischen Faden 14 bis an das proximale Ende 26 schieben lässt und dort gekrimpt werden kann, um den chirurgischen Faden festzulegen.

## Patentansprüche

1. Elektrodenfixierhülse (12) zur Fixierung einer Elektrodenleitung (16) an biologischem Gewebe, umfassend wenigstens einen Hülsenkörper (18), der wenigstens anteilig elastische Eigenschaften aufweist und der wenigstens einen Elektrodenführungsbereich (20) umfasst, wobei der Hülsenkörper (18) und der Elektrodenführungsbereich (20) eine gemeinsame Längsachse (L) aufweisen,
**dadurch gekennzeichnet, dass**
der Hülsenkörper (18) wenigstens einen Fadengang (24) aufweist, der zumindest abschnittsweise entlang der gemeinsamen Längsachse (L) und helixartig um den Elektrodenführungsbereich (20) herum verläuft.

2. Elektrodenfixierhülse (12) nach Anspruch 1, **dadurch gekennzeichnet, dass** der Fadengang (24) sich entlang der gemeinsamen Längsachse (L) von einem proximalen Ende (26) des Hülsenkörpers (18) bis zu einem distalen Ende (28) des Hülsenkörpers (18) erstreckt.

3. Elektrodenfixierhülse (12) nach Anspruch 1 oder 2, **dadurch gekennzeichnet, dass** der Fadengang (24) in einem konstanten radialen Abstand (R) von der gemeinsamen Längsachse (L) um den Elektrodenführungsbereich (20) herum verläuft.

4. Elektrodenfixierhülse (12) nach Anspruch 1 oder 2, **dadurch gekennzeichnet, dass** der Fadengang (24) in einem wenigstens einmal variierenden radialen Abstand (R) von der gemeinsamen Längsachse (L) um den Elektrodenführungsbereich (20) herum verläuft.

5. Elektrodenfixierhülse (12) nach einem der vorhergehenden Ansprüche, **dadurch gekennzeichnet, dass** der Elektrodenführungsbereich (20) eine zylindrische Durchgangsbohrung (22) entlang der gemeinsamen Längsachse (L) umfasst, die einen Durchmesser (D) von 1 mm bis 3 mm aufweist.

6. Elektrodenfixierhülse (12) nach Anspruch 3 oder 4, jeweils in Verbindung mit Anspruch 5, **dadurch gekennzeichnet, dass** der radiale Abstand (R) derart gewählt ist, dass sich zwischen einer inneren Mantelfläche (30) der zylindrischen Durchgangsbohrung (22) und dem Fadengang (24) ein konstanter oder variierender radialer Teilabstand (r) von 0,5 mm bis 3 mm ergibt.

7. Elektrodenfixierhülse (12) nach einem der vorhergehenden Ansprüche, **dadurch gekennzeichnet, dass** der Fadengang (24) einen Gangdurchmesser (d) von 0,015 mm bis 1,5 mm und/oder eine Gangsteigung (s) von 0,05 mm bis 3,02 mm pro Umlauf des Fadengangs (24) um die gemeinsame Längsachse (L) aufweist.

8. Elektrodenfixierhülse (12) nach einem der vorhergehenden Ansprüche, **dadurch gekennzeichnet, dass** der Elektrodenführungsbereich (20) einen Reibungskoeffizienten (µ) von wenigstens 0,8 gegenüber Kunststoffen aufweist.

9. System (10), wenigstens umfassend:
- eine Elektrodenfixierhülse (12) nach einem der Ansprüche 1 bis 8; und
- einen chirurgischen Faden (14).

10. System (10) nach Anspruch 9, **dadurch gekennzeichnet, dass** ein Gangdurchmesser (d) und/oder eine Gangsteigung (s) eines Fadengangs (24) eines Hülsenkörpers (18) der Elektrodenfixierhülse (12) und ein Fadendurchmesser (d') des chirurgischen Fadens (14) und/oder deren Werkstoffe derart aufeinander abgestimmt sind, dass der chirurgische Faden (14) unter Gleitreibungsbedingungen in dem Fadengang (24) beweglich ist, wenn eine Zugkraft (F_{z}) von 2 N bis 50 N auf den chirurgischen Faden (14) wirkt.

11. System (10) nach Anspruch 9 oder 10, **dadurch gekennzeichnet, dass** der chirurgische Faden (14) an einem distalen Ende (28) eines Hülsenkörpers (18) der Elektrodenfixierhülse (12) fest mit dieser verbunden ist.

12. System (10) nach einem der Ansprüche 9 bis 11, weiterhin umfassend wenigstens eine Klemmhülse (34) zur Festlegung des chirurgischen Fadens (14) an einem proximalen Ende (26) eines Hülsenkörpers (18) der Elektrodenfixierhülse (12).

13. System (10) nach einem der Ansprüche 9 bis 12, weiterhin umfassend wenigstens eine Elektrodenleitung (16).

14. System (10) nach Anspruch 13, **dadurch gekennzeichnet, dass** ein Reibungskoeffizient (µ) zwischen einem Elektrodenführungsbereich (20) eines Hülsenkörpers (18) der Elektrodenfixierhülse (12) und der Elektrodenleitung (16) in einem Bereich von wenigstens 0,8 liegt.

15. System (10) nach Anspruch 13 oder 14, **dadurch gekennzeichnet, dass** der chirurgische Faden (14), die Elektrodenfixierhülse (12) und die Elektrodenleitung (16) derart aufeinander abgestimmt sind, dass unter Wirksamkeit einer Zugkraft (F_{z}) von 2 N bis 50 N auf den chirurgischen Faden (14) ein Haftschluss zwischen einem Elektrodenführungsbereich (20) der Elektrodenfixierhülse (12) und der Elektrodenleitung (16) herstellbar ist, der eine Haftreibungskraft (Fₕ) von 2 N bis 50 N zu übertragen vermag.
